(19) Europäisches Patentamt
European Patent Office
Office européen des brevets

(11) EP 3 871 764 A1

(12) EUROPEAN PATENT APPLICATION

(43) Date of publication:
**01.09.2021 Bulletin 2021/35**

(21) Application number: **20305187.5**

(22) Date of filing: **26.02.2020**

(51) Int Cl.:
***B01J 13/16*** (2006.01) ***A61K 8/11*** (2006.01)
***A61K 9/48*** (2006.01) ***A61Q 19/10*** (2006.01)
***C11D 3/50*** (2006.01) ***C11D 17/00*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Takasago International Corporation**
**Tokyo 144-8721 (JP)**

(72) Inventors:
- **BETT, William**
**Paris (FR)**
- **RIBAUT, Tiphaine**
**Paris (FR)**

(74) Representative: **Cabinet Beau de Loménie**
**158, rue de l'Université**
**75340 Paris Cedex 07 (FR)**

# (54) AQUEOUS DISPERSION OF MICROCAPSULES, AND USES THEREOF

(57) The invention relates to an aqueous dispersion of microcapsules, said microcapsules comprising a core and a shell, wherein said shell is formed of the reaction product of (i) at least one α,β-unsaturated carbonyl compound, (ii) at least one multifunctional amine, and (iii) solid colloidal particles. The invention also relates to a process for the manufacture of such an aqueous dispersion.

EP 3 871 764 A1

## Description

### Field of the Invention

The present invention relates to an aqueous dispersion of microcapsules which comprise a hydrophobic core enclosed within a shell, a process for the manufacture of an aqueous dispersion of microcapsules as well as consumer products (such as laundry products, personal care products and cosmetic products) containing that aqueous dispersion of microcapsules.

### Background

There is continuing interest in the preparation of fragrance compositions and in the use of such compositions in consumer products. Consumers are in growing demand for perfumed products, which provide a perception of e.g. freshness and/or stimulation, which in turn reinforces consumer confidence in the efficacy of such products. Fragrance compositions can be incorporated into end products in free form and/or in encapsulated form. Microencapsulation represents a common solution to protect (e.g. upon storage) and control the delivery of hydrophobic materials such as fragrances. General descriptions and methods of preparation of microcapsules can be found in "MICROENCAPSULATION: Methods and Industrial Applications Edited by Benita and Simon (Marcel Dekker, Inc. 1996)". Microcapsules are also described in Kirk Othmer's Encyclopaedia of Chemical Technology 5th edition. Microcapsules can be formed by a variety of techniques. Mechanically formed capsules can be formed by means, such as spray chilling, by compression of solids or by spray drying emulsions. Chemically formed capsules are produced by chemical reactions forming ionic or covalent bonds using techniques such as co-acervation, interfacial polymerisation, condensation reactions and free radical polymerisation. One type of microcapsule, referred to as a wall or shell or core shell microcapsule, comprises a generally spherical shell of water- and oil-insoluble materials, typically a network polymer material, within which fragrance or other hydrophobic material is contained.

Over the past few years there has also been a growing demand for environmentally friendly consumer products. In this context the Applicant has developed fragrance-containing microcapsules which display satisfactory olfactive properties when incorporated into consumer products, and which are biodegradable.

### Summary of the invention

The present invention relates to an aqueous dispersion of microcapsules, said microcapsules comprising a hydrophobic core and a shell, wherein said shell is formed of the reaction product of (i) at least one $\alpha,\beta$-unsaturated carbonyl compound, (ii) at least one multifunctional amine, and (iii) solid colloidal particles. The invention also relates to a product comprising the above-mentioned aqueous dispersion, and to a process for the manufacture of said aqueous dispersion.

### Description of the invention

Unless otherwise stated, all percentages are weight percentages.

Unless otherwise indicated, all chemical terms have the meanings defined by the IUPAC Compendium of Chemical Terminology 2nd Edition Compiled by A D McNaught and A Wilkinson Blackwell Scientific Publications Oxford 1997 and IUPAC Nomenclature of Organic Chemistry, published by Blackwell Scientific Publications Oxford 1993 ISBN 0632034882.

Unless otherwise indicated "(meth)acrylate" (or "(meth)acrylic") means methacrylate (or methacrylic) and/or acrylate (or acrylic). For example, it means methacrylate (or methacrylic). For example it means acrylate (or acrylic). For example it means methacrylate (or methacrylic) and acrylate (or acrylic).

Unless otherwise indicated, room temperature is from 20 to 25°C, and preferably 20°C.

Certain substances, notably perfumery molecules, may exist as distinct isomers (or as mixture of distinct isomers). Hereinafter, they may be identified also by means of their CAS number. In these cases, the CAS number of a single isomer is reported. However, and unless otherwise indicated, the reference shall be understood to cover all existing isomers.

In the context of the application the terms "dispersion" and "slurry" can be used interchangeably.

In the context of the application the adjective "multifunctional", when applied to a compound, indicates that the compound comprises at least two identical reactive functions (such as carbonyl, amino, (meth)acrylate, (meth)acrylamide).

In the context of the application, the various embodiments described in the various aspects of the invention can be combined.

In a first aspect, the present invention relates to an an aqueous dispersion of microcapsules, said microcapsules comprising a hydrophobic core and a shell, wherein said shell is formed of the reaction product of (i) at least one $\alpha,\beta$-unsaturated carbonyl compound, (ii) at least one multifunctional amine, and (iii) solid colloidal particles.

Multifunctional amines useful to form the shell of the microcapsules are any suitable compounds bearing at least two amino groups, such as two, three, four or five amino groups, and may be linear or branched aliphatic amines or aromatic amines, or amino-functionalized aliphatic or aromatic heterocycles.

In one embodiment, the multifunctional amine used to form the shell of the microcapsules is a compound of formula (1):

$$R_1HN\text{-}A\text{-}NHR_2 \qquad (1)$$

where A is a direct bond, a branched or linear $(C_2\text{-}C_8)$alkylene, a $(C_6\text{-}C_{12})$arylene; a group $-(CH_2)_m-(C_6\text{-}C_{12})$arylene$-(CH_2)_m-$ where m is an integer from 1 to 3, or a group $-CH_2-(CH_2\text{-}NH\text{-}CH_2)_v-CH_2-$ in which v is an integer from 1 to 4; and $R_1$ and $R_2$ are each independently H or a $(C_1\text{-}C_4)$alkyl, or $R_1$ and $R_2$ are bound together to form o 6- or 7-membered ring. In a preferred embodiment A is a branched or linear $(C_2\text{-}C_8)$alkylene, or a group $-(CH_2)_m-(C_6\text{-}C_{12})$arylene$-(CH_2)_m-$. In a further preferred embodiment, the $(C_6\text{-}C_{12})$arylene is a phenylene.

In one embodiment, the multifunctional amine used to form the shell of the microcapsules is a compound of formula (2):

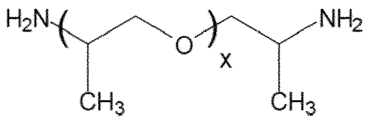
(2)

where x varies from 2 to 75.

In one embodiment, the multifunctional amine used to form the shell of the microcapsules is a compound of formula (3):

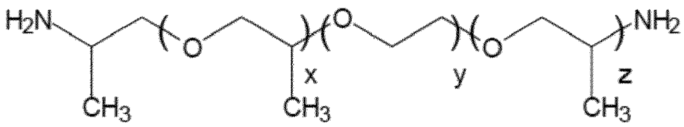
(3)

where x varies from 2 to 45 and (x+z) varies from 1 to 8.

In one embodiment, the multifunctional amine used to form the shell of the microcapsules is a compound of formula (4):

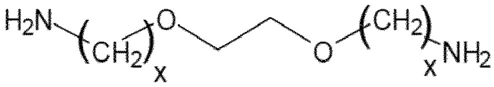
(4)

where x varies from 2 to 4.

In one embodiment, the multifunctional amine used to form the shell of the microcapsules is a compound of formula (5):

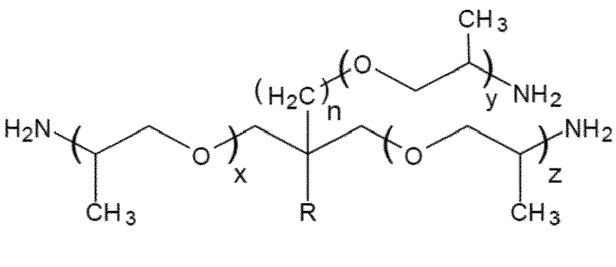
(5)

where R is H or $C_2H_5$, n is 0 or 1 and (x+y+z) varies from 5 to 90.

In one embodiment, the multifunctional amine used to form the shell of the microcapsules is selected from 1,3-diaminobenzene, 1,4-diaminobenzene, 1,3-Bis(aminomethyl)benzene, 1,4-Bis(aminomethyl)benzene, triethylene tetramine, tetraethylene pentamine, a compound of formula (2) where x =2 or 3, and a compound of formula (5) where (x+y+z) varies from 5 to 10.

$\alpha,\beta$-unsaturated carbonyl compounds useful to form the shell of the microcapsules are any suitable compounds comprising a carbonyl group and at least another (electron-withdrawing) group capable of reacting with an amino group of the multifunctional amine. In one embodiment, the $\alpha,\beta$-unsaturated carbonyl compound used to form the shell of the microcapsules is an $\alpha,\beta$-unsaturated ester, an anhydride, a multifunctional acrylamide or a multifunctional methacrylamide. Suitable anhydrides include methacrylic anhydride and acrylic anhydride. Suitable multifunctional (meth)acrylamides include N,N'-methylenebisacrylamide and N,N'-methylenebismethacrylamide.

In one embodiment, the $\alpha,\beta$-unsaturated carbonyl compound used to form the shell of the microcapsules is a multifunctional (meth)acrylate. Suitable multifunctional (meth)acrylates include di(meth)acrylates, tri(meth)acrylates, tetra(meth)acrylates, penta(meth)acrylates and hexa(meth)acrylates. In a preferred embodiment, the multifunctional (meth)acrylate is an ester of (meth)acrylic acid with a linear or branched, alicyclic, aromatic or heterocyclic ($C_2$-$C_{24}$)alcohol, preferably a ($C_2$-$C_{12}$)alcohol, or with a ($C_2$-$C_{24}$)polyethylene glycol, preferably a ($C_2$-$C_{12}$)polyethylene glycol. Suitable alcohols include those having a number average molecular weight of up to about 6000. Suitable polyethylene glycols include those having a number average molecular weight of up to about 7500.

In yet a preferred embodiment, the multifunctional (meth)acrylate is selected from 1,4-butylene glycol diacrylate, ethylene glycol diacrylate, diethylene glycol diacrylate, triethylene glycol diacrylate, neopentylglycol diacrylate, triglycerol diacrylate, 1,6-hexane diol diacrylate, trimethylolpropane triacrylate, trimethylolpropane ethoxylate triacrylate, trimethylolpropane propoxylate triacrylate, pentaerythritol triacrylate, tris-2-hydroxyethyl isocyanurate triacrylate, glycerol triacrylate, pentaerythritol tetraacrylate, di-trimethylolpropane tetraacrylate, dipentaerythritol pentaacrylate, dipentaerythritol hexaacrylate, 1,3-butylene glycol dimethacrylate, 1,4-butylene glycol dimethacrylate, 1,2-propylene glycol dimethacrylate, 1,3-propylene glycol dimethacrylate, ethylene glycol dimethacrylate, diethylene glycol dimethacrylate, glycerol dimethacrylate, 1,6-hexane diol dimethacrylate, bisphenol A dimethacrylate, bisphenol A ethoxylate dimethacrylate, pentaerythritol trimethacrylate, glycerol trimethacrylate, trimethylolpropane trimethacrylate, Tris-2-hydroxyethyl isocyanurate trimethacrylate, ethoxylated pentaerythritol tetramethacrylate, Tris[2-(acryloyloxy)ethyl]isocyanurate, 2-[4,6-bis(2-propenoyloxyethyl)-1,3,5-triazin-2-yl]ethyl prop-2-enoate, and mixtures thereof. In yet a preferred embodiment, the multifunctional (meth)acrylate is selected from 1,4-butylene glycol diacrylate, ethylene glycol diacrylate, trimethylolpropane triacrylate, pentaerythritol triacrylate, glycerol triacrylate, pentaerythritol tetraacrylate, di-trimethylolpropane tetraacrylate, 1,4-butylene glycol dimethacrylate, ethylene glycol dimethacrylate, trimethylolpropane trimethacrylate, glycerol trimethacrylate, and mixtures thereof.

The microcapsule shell comprises solid colloidal particles (also known as particulate colloids). In one embodiment, the solid colloidal particles have an average particle size comprised between 5nm and 1$\mu$m as measured by dynamic light scattering (DLS). Suitable solid colloidal particles for use within the framework of the present invention include particles of silica, quartz, glass, aluminum (AlO(OH)), lithium magnesium sodium silicates, alumino-silicates (e.g. clays), silicon, copper, tin (SnO), talc, inorganic oxides or hydroxides (e.g. $Fe_2O_3$, $TiO_2$, $Cr_2O_3$), steel, iron, asbestos, nickel, zinc, lead, marble, chalk ($CaCO_3$), gypsum ($CaSO_4$), barytes (e.g. $BaSO_4$), graphite, carbon black, nanocellulose as well as particles extracted from apple juice (INCI name: pyrus malus). In one embodiment, the solid colloidal particles are selected from particles of silica, particles of lithium magnesium sodium silicates, particles of nanocellulose and particles extracted from apple juice. In a preferred embodiment, the solid colloidal particles are silica particles.

Solid colloidal particles suitable for the present invention may or may not be surface modified. Examples of surface modification include chemical treatments to increase or decrease particles hydrophobicity. Alternatively, surface modifying agents can be adsorbed onto particles surface to impart appropriate surface active properties. Alternatively, particles may be modified by means of coupling agents which improve the compatibility between the particles and the microcapsule shell. Techniques to modify particle surfaces are discussed for example in "Nanoparticle Technology handbook" 1st edition, year 2007, Application 41 (pages 593-596) "Surface modification of inorganic nanoparticles by organic functional groups". Modified (as well as non-modified) solid colloidal particles are commercially available.

Examples of suitable colloidal silicas may be dry fumed silicas (such as the range of products marketed under the tradename Aerosil®) or aqueous colloidal silica dispersions (such as the range of products marketed under the tradename Ludox®). Dry silica particles may be fumed silica particles or condensed silica particles. Fumed silicas are particularly adapted for stabilizing emulsions with droplet sizes in the range of 10 $\mu$m to 100 $\mu$m. For larger droplets, colloidal silicas might be more appropriate. Suitable grades of fumed silica are Aerosil® 200 (a hydrophilic fumed silica with a specific surface area of 200 $m^2/g$) and Aerosil® R816 having a BET surface area of 190 $\pm$ 20$m^2/g$ and an average primary particle size of about 12 nm, both available from Evonik.

The use of solid colloidal particles as presently defined has been found to allow satisfactory and effective microencapsulation of desirably high fragrance loadings while maintaining a suitable quality of the resulting dispersion

(e.g., good processability of the dispersion). This allows encapsulating more fragrance than usual while not being confronted with insurmountable manufacturing hurdles or having to tolerate a non-ideal dispersion quality. These results are achievable without relying on complex and expensive manufacturing techniques.

The dispersion may comprise, for example essentially consist of, a water-based liquid medium (i.e. the dispersing medium) and a plurality of microcapsules dispersed in the medium. Traces of other ingredients used in the manufacturing process (such as unreacted monomers) may also be present.

In one embodiment, the water-based liquid medium comprises, for example essentially consists of, water, such as deionized water.

In one embodiment, the hydrophobic core of the microcapsules comprises, such as essentially consists of, such as consists of, a fragrance, which in that case typically represents from 20% to 45%, such as at least 25%, for example at least 30% or at least 33% and no more than 40%, for example no more than 35% by weight of the weight of the dispersion.

In one embodiment, the microcapsules as presently disclosed have a shell thickness from about 80 nm to about 800 nm, such as from about 150 nm to about 700 nm, for example from about 180 nm to about 500 nm, when dried and opened and observed with a scanning electron microscope.

In one embodiment, when the core of the microcapsules as presently disclosed comprise a fragrance, the fragrance-to-shell weight ratio is from about 50:1 to about 1:1, such as from about 30:1 to about 1:1, or from about 20:1 to about 1:1, for example from about 10:1 to about 1:1.

In one embodiment, the microcapsules presently disclosed are substantially spherical.

In one embodiment, the microcapsules presently disclosed have a median volume diameter (D(v;0.5) value) equal to or greater than 1.0 micrometer (1.0 μm), for example equal to or greater than 7.5 μm, equal to or greater than 10 μm, equal to or greater than 15 μm, equal to or greater than 20 μm, or equal to or greater than 25 μm. In a further embodiment, the microcapsules presently disclosed have a median volume diameter equal to or less than 250 micrometers (μm), for example equal to or less than 100 μm, equal to or less than 65 μm, equal to or less than 50 μm, equal to or less than 45 μm, or equal to or less than 40 μm. In a further embodiment, the microcapsule presently disclosed have a median volume diameter from 7.5 micrometers (7.5 μm) to 60 microns (60 μm), or from 7.5 μm to 50 μm, or from 10 μm to 50 μm, or from 7.5 μm to 45 μm, or from 10 μm to 45 μm, or from 15 μm to 45 μm, or from 15 μm to 40 μm, or from 20 μm to 45 μm, or from 25 μm to 45 μm, or from 25 μm to 40 μm, or from 25 μm to 35 μm.

The microcapsule median volume diameter is measured by light scattering using for example a Horiba® or a Malvern® Laser scattering particle Size Distribution analyzer or an equivalent instrument working on the principle of low angle laser light scattering (LALLS) following the general guidelines set out in ISO 13320 "Particle Size Analysis - Laser Diffraction Methods".

As indicated above the hydrophobic core of the microcapsules can comprise a fragrance. A fragrance comprises at least one and preferably a mixture of two or more olfactively active (i.e. odoriferous) compounds typically but not necessarily providing a pleasant smell. The fragrance thus typically comprises at least one, such as at least two, such as at least five, or at least eight distinct fragrance compounds. It can comprise highly complex mixtures of fragrance compounds, chosen to provide any desired odour. In the context of the present invention the term "fragrance" is intended to be synonymous with "perfume". Fragrance compounds typically used in the field of perfumery and suitable for the purposes of the present invention are described more fully in S. Arctander, Perfume Flavors and Chemicals 1969, Vols. I and II, Montclair, N. J. and in The Merck Index, 8th edition, Merck & Co., Inc. Rahway, N. J. The term "fragrance compound" encompasses naturally occurring as well as synthetic materials known for use in perfumes, as well as animal oils. A fragrance compound can also be any natural oil or extract used in a fragrance composition. Natural oils and extracts are described in The Essential Oils by E Guenther published in 1949 by Van Nostrand and may include extracts, pressings, collection of exudates, and distillates from any part of suitable plants: roots, rhizomes, bulbs, corms, stem, bark, heartwood, leaves, flowers, seeds and fruit. Examples of such extracts and distillates include citrus fruit oils such as orange, mandarin, grapefruit, lime or lemon oils, tree oils such as pine, or cedar wood, herb oils such as peppermint, thyme, lavender, basil, rosemary, clove or flower extracts such as rose, jasmine, muguet, or geranium oil.

In one embodiment, each fragrance compound has a molecular weight greater than 100 g/mol, preferably greater than 120 g/mol and lower than 325 g/mol, preferably lower than 300 g/mol. In a further embodiment each fragrance compound has a boiling point in the range 80-400°C, such as in the range 100-350°C, when measured at 760 mm Hg.

In a preferred embodiment, the fragrance compounds can advantageously be selected from the following list:

- $C_8$-$C_{18}$ hydrocarbons, preferably delta-3-carene, alpha-pinene, beta-pinene, alpha-terpinene, gamma-terpinene, p-cymene, bisabolene, camphene, caryophyllene, cedrene, farnesene, limonene, longifolene, myrcene, ocimene, valencene, (E,Z)-1,3,5-undecatriene;
- $C_2$-$C_{18}$ aliphatic alcohols, preferably hexanol, octanol, 3-octanol, 2,6-dimethylheptanol, 2-methylheptanol, 2-methyloctanol, (E)-3-hexenol, (E) and (Z)-3-hexenol, 1-octen-3-ol, mixtures of 3,4,5,6,6-pentamethyl-3/4-hepten-2-ol and 3,5,6,6-tetramethyl-4-methyleneheptan-2-ol, (E,Z)-2,6-nonadienol, 3,7-dimethyl-7-methoxyoctan-2-ol, 9-decenol,

10-undecenol, 4-methyl-3-decen-5-ol;

- $C_2$-$C_{18}$ aliphatic aldehydes and their acetals, preferably hexanal, heptanal, octanal, nonanal, decanal, undecanal, dodecanal, tridecanal, 2-methyloctanal, 2-methylnonanal, (E)-2-hexenal, (Z)-4-heptenal, 2,6-dimethyl-5-heptenal, 10-undecenal, (E)-4-decenal, 2-dodecenal, 2,6,10-trimethyl-5,9-undecadienal, heptanal diethyl acetal, 1,1-dimethoxy-2,2,5-trimethyl-4-hexene, citronellyl oxyacetaldehyde;
- $C_3$-$C_{18}$ aliphatic ketones and oximes thereof, preferably 2-heptanone, 2-octanone, 3-octanone, 2-nonanone, 5-methyl-3-heptanone, 5-methyl-3-heptanone oxime, 2,4,4,7-tetramethyl-6-octen-3-one;
- $C_2$-$C_{18}$ aliphatic sulphur-containing compounds, preferably 3-methylthiohexanol, 3-methylthiohexyl acetate, 3-mercaptohexanol, 3-mercaptohexyl acetate, 3-mercaptohexyl butyrate, 3-acetylthiohexyl acetate, 1-menthene-8-thiol;
- $C_2$-$C_{18}$ aliphatic nitrile-containing compounds, preferably 2-nonenenitrile, 2-tridecenenenitrile, 2,12-tridecenenenitrile, 3,7-dimethyl-2,6-octadienenitrile, 3,7-dimethyl-6-octenenitrile;
- $C_2$-$C_{18}$ aliphatic carboxylic acids and esters thereof, preferably (E)- and (Z)-3-hexenyl formate, ethyl acetoacetate, isoamyl acetate, hexyl acetate, 3,5,5-trimethylhexyl acetate, 3-methyl-2-butenyl acetate, (E)-2-hexenyl acetate, (E)- and (Z)-3-hexenyl acetate, octyl acetate, 3-octyl acetate, 1-octen-3-yl acetate, ethyl butyrate, butyl butyrate, isoamyl butyrate, hexyl butyrate, (E)- and (Z)-3-hexenyl isobutyrate, hexyl crotonate, ethyl isovalerate, ethyl 2-methylpentanoate, ethyl hexanoate, allyl hexanoate, ethyl heptanoate, allyl heptanoate, ethyl octanoate, ethyl (E,Z)-2,4-decadienoate, methyl 2-octynoate, methyl 2-nonynoate, allyl-2-isoamyloxyacetate, methyl-3,7-dimethyl-2,6-octadienoate;
- $C_4$-$C_{18}$ acyclic terpene alcohols, preferably citronellol, geraniol, nerol, linalool, lavandulol, nerolidol, farnesol, tetrahydrolinalool, tetrahydrogeraniol, 2,6-dimethyl-7-octen-2-ol, 2,6-dimethyloctan-2-ol, 2-methyl-6-methylene-7-octen-2-ol, 2,6-dimethyl-5,7-octadien-2-ol, 2,6-dimethyl-3,5-octadien-2-ol, 3,7-dimethyl-4,6-octadien-3-ol, 3,7-dimethyl-1,5,7-octatrien-3-ol, 2,6-dimethyl-2,5,7-octatrien-1-ol;
- $C_4$-$C_{18}$ acyclic terpene aldehydes and ketones, preferably geranial, neral, citronellal, 7-hydroxy-3,7-dimethyloctanal, 7-methoxy-3,7-dimethyloctanal, 2,6,10-trimethyl-9-undecenal, geranylacetone, and the dimethyl and diethyl acetals of geranial, neral, 7-hydroxy-3,7-dimethyloctanal;
- $C_4$-$C_{18}$ cyclic terpene alcohols, preferably alpha-terpineol, terpineol-4, menthan-8-ol, menthan-1-ol, menthan-7-ol, borneol, isoborneol, linalool oxide, nopol, cedrol, ambrinol, vetiverol, guaiol;
- $C_4$-$C_{18}$ cyclic terpene aldehydes and ketones, preferably fenchone, alpha-ionone, beta-ionone, alpha-n-methylionone, beta-n-methylionone, alpha-isomethylionone, beta-isomethylionone, alpha-irone, alpha-damascone, beta-damascone, beta-damascenone, delta-damascone, gamma-damascone, 1-(2,4,4-trimethyl-2-cyclohexen-1-yl)-2-buten-1-one, 1,3,4,6,7,8a-hexahydro-1,1,5,5-tetramethyl-2H-2,4a-methanonaphthalen-8(5H)-one, nootkatone, dihydronootkatone, alpha-sinensal, beta-sinensal, methyl cedryl ketone;
- $C_4$-$C_{18}$ cyclic alcohols, preferably 4-tert-butylcyclohexanol, 3,3,5-trimethylcyclohexanol, 3-isocamphylcyclohexanol, 2,6,9-trimethyl-Z2,Z5,E9-cyclododecatrien-1-ol, 2-isobutyl-4-methyltetrahydro-2H-pyran-4-ol;
- $C_4$-$C_{18}$ cycloaliphatic alcohols, preferably alpha-3,3-trimethylcyclohexylmethanol, 2-methyl-4-(2,2,3-trimethyl-3-cyclopent-1-yl)butanol, 2-methyl-4-(2,2,3-trimethyl-3-cyclopent-1-yl)-2-buten-1-ol, 2-ethyl-4-(2,2,3-trimethyl-3-cyclopent-1-yl)-2-buten-1-ol, 3-methyl-5-(2,2,3-trimethyl-3-cyclopent-1-yl)-pentan-2-ol, 3-methyl-5-(2,2,3-trimethyl-3-cyclopent-1-yl)-4-penten-2-ol, 3,3-dimethyl-5-(2,2,3-trimethyl-3-cyclopent-1-yl)-4-penten-2-ol, 1-(2,2,6-trimethylcyclohexyl)pentan-3-ol, 1-(2,2,6-trimethylcyclohexyl)hexan-3-ol;
- $C_4$-$C_{18}$ cyclic and cycloaliphatic ethers, preferably cedryl methyl ether, cyclododecyl methyl ether, (ethoxymethoxy)cyclododecane, alpha-cedrene epoxide, 3a,6,6,9a-tetramethyl-dodecahydronaphtho[2,1-b]furan, 3a-ethyl-6,6,9a-trimethyldodecahydro-naphtho[2,1-b]furan, 1,5,9-trimethyl-13-oxabicyclo[10.1.0]trideca-4,8-diene, rose oxide, 2-(2,4-dimethyl-3-cyclohexen-1-yl)-5-methyl-5-(1-methylpropyl)-1,3-dioxane;
- $C_4$-$C_{18}$ cyclic ketones, preferably 4-tert-butylcyclohexanone, 2,2,5-trimethyl-5-pentylcyclopentanone, 2-heptylcyclopentanone, 2-pentylcyclopentanone, 2-hydroxy-3-methyl-2-cyclopenten-1-one, 3-methyl-cis-2-penten-1-yl-2-cyclopenten-1-one, 3-methyl-2-pentyl-2-cyclopenten-1-one, 3-methyl-4-cyclopentadecenone, 3-methyl-5-cyclopentadecenone, 3-methylcyclopentadecanone, 4-(1-ethoxyvinyl)-3,3,5,5-tetramethylcyclohexanone, 4-tert-pentylcyclohexanone, 5-cyclohexadecen-1-one, 6,7-dihydro-1,1,2,3,3-pentamethyl-4(5H)-indanone, 9-cycloheptadecen-1-one, cyclopentadecanone, cyclohexadecanone;
- $C_4$-$C_{18}$ cycloaliphatic aldehydes, preferably 2,4-dimethyl-3-cyclohexenecarbaldehyde, 2-methyl-4-(2,2,6-trimethylcyclohexen-1-yl)-2-butenal, 4-(4-hydroxy-4-methylpentyl)-3-cyclohexenecarbaldehyde, 4-(4-methyl-3-penten-1-yl)-3-cyclohexenecarbaldehyde;
- $C_4$-$C_{18}$ cycloaliphatic ketones, preferably 1-(3,3-dimethylcyclohexyl)-4-penten-1-one, 1-(5,5-dimethyl-1-cyclohexen-1-yl)-4-penten-1-one, 2,3,8,8-tetramethyl-1,2,3,4,5,6,7,8-octahydro-2-naphthalenyl methyl ketone, methyl-2,6,10-trimethyl-2,5,9-cyclododecatrienyl ketone, tert-butyl(2,4-dimethyl-3-cyclohexen-1-yl)ketone;
- esters of cyclic alcohols in $C_4$-$C_{18}$, preferably 2-tert-butylcyclohexyl acetate, 4-tert-butyl-cyclohexyl acetate, 2-tert-pentylcyclohexyl acetate, 4-tert-pentylcyclohexyl acetate, decahydro-2-naphthyl acetate, 3-pentyltetrahydro-2H-pyran-4-yl acetate, decahydro-2,5,5,8a-tetramethyl-2-naphthyl acetate, 4,7-methano-3a,4,5,6,7,7a-hexahydro-5 or

6-indenyl acetate, 4,7-methano-3a,4,5,6,7,7a-hexahydro-5 or 6-indenyl propionate, 4,7-methano-3a,4,5,6,7,7a-hexahydro-5 or 6-indenyl isobutyrate, 4,7-methanooctahydro-5 or 6-indenyl acetate;

- esters of cycloaliphatic carboxylic acids in $C_4$-$C_{18}$, preferably allyl 3-cyclohexylpropionate, allyl cyclohexyloxyacetate, methyl dihydrojasmonate, methyl jasmonate, methyl 2-hexyl-3-oxocyclopentanecarboxylate, ethyl 2-ethyl-6,6-dimethyl-2-cyclohexenecarboxylate, ethyl 2,3,6,6-tetramethyl-2-cyclohexenecarboxylate, ethyl 2-methyl-1,3-dioxolane-2-acetate;
- $C_4$-$C_{18}$ aromatic hydrocarbons, preferably styrene and diphenylmethane;
- $C_4$-$C_{18}$ araliphatic alcohols, preferably benzyl alcohol, 1-phenylethyl alcohol, 2-phenylethyl alcohol, 3-phenylpropanol, 2-phenylpropanol, 2-phenoxyethanol, 2,2-dimethyl-3-phenylpropanol, 2,2-dimethyl-3-(3-methylphenyl)propanol, 1,1-dimethyl-2-phenylethyl alcohol, 1,1-dimethyl-3-phenylpropanol, 1-ethyl-1-methyl-3-phenylpropanol, 2-methyl-5-phenylpentanol, 3-methyl-5-phenylpentanol, 3-phenyl-2-propen-1-ol, 4-methoxybenzyl alcohol, 1-(4-isopropylphenyl)ethanol;
- esters of araliphatic alcohols in $C_4$-$C_{18}$ and aliphatic carboxylic acids in $C_4$-$C_{18}$, preferably benzyl acetate, benzyl propionate, benzyl isobutyrate, benzyl isovalerate, 2-phenylethyl acetate, 2-phenylethyl propionate, 2-phenylethyl isobutyrate, 2-phenylethyl isovalerate, 1-phenylethyl acetate, alpha-trichloromethylbenzyl acetate, alpha,alpha-dimethylphenylethyl acetate, alpha,alpha-dimethylphenylethyl butyrate, cinnamyl acetate, 2-phenoxyethyl isobutyrate, 4-methoxybenzyl acetate;
- $C_2$-$C_{18}$ araliphatic ethers, preferably 2-phenylethyl methyl ether, 2-phenylethyl isoamyl ether, 2-phenylethyl 1-ethoxyethyl ether, phenylacetaldehyde dimethyl acetal, phenylacetaldehyde diethyl acetal, hydratropaldehyde dimethyl acetal, phenylacetaldehyde glycerol acetal, 2,4,6-trimethyl-4-phenyl-1,3-dioxane, 4,4a,5,9b-tetrahydroindeno[1,2-d]-m-dioxin, 4,4a,5,9b-tetrahydro-2,4-dimethylindeno[1,2-d]-m-dioxin;
- $C_4$-$C_{18}$ aromatic and araliphatic aldehydes, preferably benzaldehyde, phenylacetaldehyde, 3-phenylpropanal, hydratropaldehyde, 4-methylbenzaldehyde, 4-methylphenylacetaldehyde, 3-(4-ethylphenyl)-2,2-dimethylpropanal, 2-methyl-3-(4-isopropylphenyl)propanal, 2-methyl-3-(4-tert.-butylphenyl)propanal, 3-(4-tert-butylphenyl)propanal, cinnamaldehyde, alpha-butylcinnamaldehyde, alpha-amylcinnamaldehyde, alpha-hexylcinnamaldehyde, 3-methyl-5-phenylpentanal, 4-methoxybenzaldehyde, 4-hydroxy-3-methoxybenzaldehyde, 4-hydroxy-3-ethoxybenzaldehyde, 3,4-methylenedioxybenzaldehyde, 3,4-dimethoxybenzaldehyde, 2-methyl-3-(4-methoxyphenyl)propanal, 2-methyl-3-(4-methylenedioxyphenyl)propanal;
- $C_4$-$C_{18}$ aromatic and araliphatic ketones, preferably acetophenone, 4-methylacetophenone, 4-methoxyacetophenone, 4-tert-butyl-2,6-dimethylacetophenone, 4-phenyl-2-butanone, 4-(4-hydroxyphenyl)-2-butanone, 1-(2-naphthalenyl)ethanone, benzophenone, 1,1,2,3,3,6-hexamethyl-5-indanyl methyl ketone, 6-tert-butyl-1,1-dimethyl-4-indanyl methyl ketone, 1-[2,3-dihydro-1,1,2,6-tetramethyl-3-(1-methylethyl)-1H-5-indenyl]ethanone, 5',6',7',8'-tetrahydro-3',5',5',6',8',8'-hexamethyl-2-acetonaphthone;
- $C_4$-$C_{18}$ aromatic and araliphatic carboxylic acids and esters thereof, preferably phenylacetic acid, methyl benzoate, ethyl benzoate, hexyl benzoate, benzyl benzoate, methyl phenylacetate, ethyl phenylacetate, geranyl phenylacetate, phenylethyl phenylacetate, methyl cinnamate, ethyl cinnamate, benzyl cinnamate, phenylethyl cinnamate, cinnamyl cinnamate, allyl phenoxyacetate, methyl salicylate, isoamyl salicylate, hexyl salicylate, cyclohexyl salicylate, cis-3-hexenyl salicylate, benzyl salicylate, phenylethyl salicylate, methyl 2,4-dihydroxy-3,6-dimethylbenzoate, ethyl 3-phenylglycidate, ethyl 3-methyl-3-phenylglycidate;
- nitrogen-containing aromatic compounds in $C_4$-$C_{18}$, preferably 2,4,6-trinitro-1,3-dimethyl-5-tert-butylbenzene, 3,5-dinitro-2,6-dimethyl-4-tert-butylacetophenone, cinnamonitrile, 5-phenyl-3-methyl-2-pentenenitrile, 5-phenyl-3-methylpentanenitrile, methyl anthranilate, methyl N-methylanthranilate, Schiff bases of methyl anthranilate with 7-hydroxy-3,7-dimethyloctanal, 2-methyl-3-(4-tert-butylphenyl)propanal, 2,4-dimethyl-3-cyclohexene-carbaldehyde, 6-isopropylquinoline, 6-isobutylquinoline, 6-sec-butylquinoline, indole, skatole, 2-methoxy-3-isopropylpyrazine, 2-isobutyl-3-methoxypyrazine;
- phenols, phenyl ethers and phenyl esters, preferably estragole, anethole, eugenol, eugenyl methyl ether, isoeugenol, isoeugenyl methyl ether, thymol, carvacrol, diphenyl ether, beta-naphthyl methyl ether, beta-naphthyl ethyl ether, beta-naphthyl isobutyl ether, 1,4-dimethoxybenzene, eugenyl acetate, 2-methoxy-4-methylphenol, 2-ethoxy-5-(1-propenyl)phenol, p-cresyl phenylacetate;
- heterocyclic compounds in $C_4$-$C_{12}$, preferably 2,5-dimethyl-4-hydroxy-2H-furan-3-one, 2-ethyl-4-hydroxy-5-methyl-2H-furan-3-one, 3-hydroxy-2-methyl-4H-pyran-4-one, 2-ethyl-3-hydroxy-4H-pyran-4-one;
- lactones in $C_4$-$C_{18}$, preferably 1,4-octanolide, 3-methyl-1,4-octanolide, 1,4-nonanolide, 1,4-decanolide, 8-decen-1,4-olide, 1,4-undecanolide, 1,4-dodecanolide, 1,5-decanolide, 1,5-dodecanolide, 1,15-pentadecanolide, cis and trans-11-pentadecen-1,15-olide, cis- and trans-12-pentadecen-1,15-olide, 1,16-hexadecanolide, 9-hexadecen-1,16-olide, 10-oxa-1,16-hexadecanolide, 11-oxa-1,16-hexadecanolide, 12-oxa-1,16-hexadecanolide, ethylene 1,12-dodecanedioate, ethylene 1,13-tridecanedioate, coumarin, 2,3-dihydrocoumarin, octahydrocoumarin.

In an especially preferred embodiment, if fragrance compounds which can act as Michael donors or acceptors

are present in the fragrance composition, the sum of all such compounds makes up at most 5 wt% of the fragrance composition.

In one embodiment, the fragrance compounds present in the fragrance do not contain ionizing functional groups, such as sulfonates, sulphates, phosphates or quaternary ammonium ions.

In one embodiment, the fragrance defined above includes one or more support materials, such as solvents or UV stabilizers. Examples of suitable solvents include hydrocarbons such as those sold under the trade name Isopar®; ethers such as those sold under the Dowanol® trade name; benzyl benzoate; isopropyl myristate; dialkyl adipates; dialkyl succinates; dialkyl glutarates such as the dimethyl esters sold under the trade name Flexisolv®; citrate esters, such as triethyl citrate and acetyl tributyl citrate; soybean methyl ester such as ME-S1885 (sold by Peter Cremer NA); diethyl phthalate; diethylene glycol monoethyl ether; 3-methoxy-3-methyl-1-butanol; dipropylene glycol; and isopropylidene glycerol sold under the Augeo® Clean Multi brand name. Examples of UV stabilisers include butyl methoxy dibenzoyl methane; bis ethylhexyloxyphenolmethoxyphenyl triazine; those sold under the Uvinol® trade name such as Uvinul D50 [bis(2,4-dihydroxyphenyl)-methanone], Parsol® 1789 (butyl methoxydibenzoylmethane); and those sold under the Tinogard® trade name.

In one embodiment, the fragrance comprise up to 60% by weight, such as up to 70% by weight, up to 80% by weight, up to 90% by weight or even 100% by weight of fragrance compounds, the remainder (if appropriate) being made up of support materials as defined above.

In one embodiment, the core of the microcapsules comprises at least 70% by weight, such as at least 80% by weight or at least 90% by weight of fragrance.

In a second aspect, the invention relates to a process for the manufacture of an aqueous dispersion as defined above. The process comprises (i) forming an oil-in-water emulsion wherein the oil phase comprises the constituent(s) of the hydrophobic core and the at least one $\alpha,\beta$-unsaturated carbonyl compound, and the aqueous phase comprises solid colloidal particles, (ii) adding a polymeric stabilizer to the emulsion, and (iii) adding an aqueous solution of the at least one multifunctional amine to the stabilized emulsion. The reaction, at the oil-water interface, of the $\alpha,\beta$-unsaturated carbonyl compound(s), the solid colloidal particles, and the multifunctional amine(s) will form the shell of the microcapsules, surrounding droplets of the hydrophobic core constituent(s).

In one embodiment, the at least one $\alpha,\beta$-unsaturated carbonyl compound and the at least one multifunctional amine are used in the above-described process in amounts such that the molar ratio of the (reactive) functions (carbonyl and other) of the $\alpha,\beta$-unsaturated carbonyl compound(s) to the amine functions of the multifunctional amine(s) is in the range from about 1:0.6 to about 1:1.4, preferably from about 1:0.7 to about 1:1.3, preferably from about 1:0.8 to about 1:1.2, wherein the molar amount of functions of each compound is calculated as follows:

Molar amount of functions in a compound = molar amount of compound/number of functions in the compound.

In one embodiment, the oil-in-water emulsion is obtained by mixing, at a temperature ranging from room temperature to about 70°C, an oil phase comprising the constituent(s) of the hydrophobic core and the at least one $\alpha,\beta$-unsaturated carbonyl compound, and an aqueous phase comprising the solid colloidal particles. Solid colloid particles are typically added in an amount from about 0.05% to about 10% by weight, preferably from about 0.05% to about 6% by weight, relative to the weight of the water phase of the oil-in-water emulsion.

In one embodiment, silane coupling agents may be used in conjunction with the solid colloidal particles to increase the compatibility between the polymer formed and the solid colloidal particles. Suitable silane coupling agents may be selected from 3-acrylamidopropyl-trimethoxysilane, acryloxymethyltrimethoxysilane, (3-acryloxypropyl)-trimethoxysilane, (3-acryloxypropyl)methyldiethoxysilane, 3-acryloxypropyl)-methyldimethoxysilane, o-(methacryloxyethyl)-N-(triethoxysilylpropyl)carbamate, methacryloxypropyl-trimethoxysilane, methacryloxypropyltriethoxysilane, o-(methacryloxyethyl)-N-(triethoxysilylpropyl)urethane, methacryloxymethyltrimethoxysilane, (methacryloxy-methyl)methyldiethoxysilane, (methacryloxymethyl)methyl dimethoxysilane, and mixtures thereof. The silane coupling agent is advantageously selected from (3-acryloxypropyl)-trimethoxysilane, methacryloxypropyltrimethoxysilane, and mixtures thereof. When present, the silane coupling agent is typically used in an amount from 50 and 300% by weight, preferably from 100 to 200% by weight, relative to the total weight of solid colloidal particles.

Once the emulsion has been formed, a polymeric stabilizer can be added, typically at a temperature between room temperature and about 70°C. Polymeric stabilizers are conventionally used to stabilize oil-in-water emulsions created by mechanical agitation.

In one embodiment, the polymeric stabilizer is selected from:

- cellulose derivatives such as hydroxyethylcellulose, carboxymethylcellulose and methylcellulose,

- polyvinylpyrrolidone,
- copolymers of N-vinylpyrrolidone,
- polyvinyl alcohols obtainable by full to partial hydrolyses of polyvinyl acetates,
- polyacrylic and/or polymethacrylic acid,
- copolymers of acrylic acid (or an ester thereof) and methacrylic acid (or an ester thereof),
- ionic colloids such as sulphonic-acid-group-containing water-soluble polymers (e.g. 2-acrylamido-2-alkylsulphonic acids and styrene sulphonic acids), and
- mixtures thereof.

In one embodiment the polymeric stabilizer has a molecular weight greater than about 5,000 g/mol, preferably greater than about 10,000 g/mol, more preferably greater than about 50,000 g/mol. In another embodiment, the polymeric stabilizer has a molecular weight less than about 150,000 g/mol, preferably less than about 100,000 g/mol.

In one embodiment, the polymeric stabilizer is a polyvinyl alcohol (PVA), which has preferably a molecular weight as defined above.

In one embodiment, the polymeric stabilizer is present in an amount from about 0.1% to about 10% by weight of the weight of the water phase of the oil-in-water emulsion.

After the emulsion has been stabilized (if required), it is heated to a temperature in the range from about 30°C to about 70°C, and an aqueous solution of the at least one polyamine is then added while keeping the temperature of the reaction in the same range. The reaction is then left in that temperature range for about 30 min to about 4h, after which time the microcapsules are formed, and the dispersion is allowed to cool to room temperature.

Optionally, deposition aids can be included to increase deposition or adhesion of the microcapsules to various surfaces such as various natural and synthetic substrates including but not limited to paper, fabric skin, hair, plastic, leather, ceramic, or other surfaces. Deposition aids can include poly (acrylamide-co-diallyldimethylammonium) chloride, poly(diallyldimethylammonium) chloride, polyethylenimine, cationic polyamine, poly[(3-methyl-1-vinylimidazolium chloride)-co-(1-vinylpyrrolidone)], copolymer of acrylic acid and diallyldimethylammonium chloride, cationic guar, guar gum, an organopolysiloxane such as described in US patent application 2015/0030557. Deposition aids can also be selected from poly(meth)acrylate, poly(ethylene-maleic anhydride), polyamine, wax, polyvinylpyrrolidone, polyvinylpyrrolidone co-polymers, polyvinylpyrrolidone-ethyl acrylate, polyvinylpyrrolidone-vinyl acrylate, polyvinylpyrrolidone methylacrylate, polyvinylpyrrolidone-vinyl acetate, polyvinyl acetal, polyvinyl butyral, polysiloxane, poly(propylene maleic anhydride), maleic anhydride derivatives, co-polymers of maleic anhydride derivatives, polyvinyl alcohol, styrenebutadiene latex, gelatin, gum Arabic, carboxymethyl cellulose, carboxymethyl hydroxyethyl cellulose, hydroxyethyl cellulose, other modified celluloses, sodium alginate, chitosan, casein, pectin, modified starch, polyvinyl acetal, polyvinyl butyral, polyvinyl methyl ether/maleic anhydride, polyvinyl pyrrolidone and its co polymers, poly(vinyl pyrrolidone/methacrylamidopropyltrimethylammonium chloride), polyvinylpyrrolidone/vinyl acetate, polyvinyl pyrrolidone/dimethylaminoethyl methacrylate, polyvinyl amines, polyvinyl formamides, polyallyl amines and copolymers of polyvinyl amines, polyvinyl formamides, and polyallyl amines and mixtures thereof. In one embodiment, the deposition aid coats the outer surface of the shell of the microcapsule.

The aqueous dispersion of microcapsules of the invention can advantageously be incorporated into a variety of consumer products.

Accordingly, a third aspect of the invention is concerned with a product comprising an aqueous dispersion of microcapsules as disclosed above. The product may be a non-edible consumer goods product, a household cleaner or laundry product, a personal care product or a cosmetic product.

Unless otherwise indicated, non-edible means non-intended for ingestion by humans or animals. This includes non-food products that may accidentally be swallowed during normal use. Notably, included within the definition of non-edible products are products for dental and oral care, such as toothpastes, mouth washes and lip balms which although not intended for ingestion may nevertheless accidentally enter the gastro-intestinal tract. The formulations and ingredients of liquid household, laundry, personal care and cosmetic products in which the dispersion of microcapsules of the invention may be used are well known to those skilled in the art, reference may be made to the following works:

- Formulating Detergents and Personal Care Products A guide to Product Development by L Ho Tan Tai, ISBN 1-893997-10-3 published by the AOCS Press
- Volume 67 of the Surfactant Science Series Liquid Detergents ISBN 0-8247-9391-9 (Marcel Dekker Inc),
- Harry's Cosmeticology published by CHS Press 8th Edn. 2000 ISBN 0820603724.

Personal care and cosmetic products include products that can be applied to the skin, hair and nails, either as leave on or rinse off product. In the context of the invention "rinse-off" means that the intended product use includes application to skin and/or hair followed by rinsing and/or wiping the product from the skin and/or hair within a few seconds to minutes of the application step. Personal care and cosmetic products include powders, creams, emulsions, lotions,

gels and oils for the skin (face, hands, feet etc), tinted bases (liquids and pastes) and liquid impregnated tissues; products for applying and removing make-up from the face and eyes; hair care products including hair tints and bleaches; products for waving, straightening, setting and fixing hair; shaving products including creams, foams mousses and depilatory products; sun bathing products and products for tanning without the sun; deodorant and antiperspirant products.

In one embodiment a personal care or cosmetic product is selected from the group consisting of a shaving aid, a shampoo, a hair-conditioner product, a leave-on-skin-care product, a skin cleansing or washing product (such as a rinse-off skin cleansing or washing product), a moist tissue and a body spray, deodorant or antiperspirant.

Shaving aids specifically include foams, gels, creams and bars (reference can be made for example to US7,069,658, US6,944,952, US6,594,904, US6,182,365, US6,185,822, US6,298,558 and US5,113,585).

Shampoos and hair conditioners specifically include two-in-one shampoos and shampoos especially formulated for dry or greasy hair or containing additives such as antidandruff agents. Hair conditioners may be rinse off or leave on hair conditioners also included are hair tonics, bleaches colorants, setting and styling products. Reference can be made for example to US 6,162,423, US 5,968,286, US 5,935,561, US 5,932,203, US 5,837,661, US 5,776,443, US 5,756,436, US 5,661,118, US 5,618,523.

Leave-on-skin-care products comprise skin washing products, moist tissues, body sprays, deodorants and antiperspirants.

Skin washing products specifically include beauty and hygiene bar soaps, shower gels, liquid soaps, body washes, exfoliating gels and pastes (reference can be made for example to US3,697,644; US4,065,398; US4,387,040).

Moist tissues (wipes) specifically include skin cleansing wipes, baby wipes, make-up removal wipes and skin refreshing wipes (reference can be made for example to US4,775,582; WO02/07701; WO2007/069214 and WO95/16474).

Body sprays, deodorants and antiperspirants specifically include sticks, liquid roll-on applicators and pressurized sprays.

Household products include hard surface cleaners such as cleaners for floors, solid work surfaces, tiled surfaces, crockery by hand or machine washing and mirrors and glass; and soft furnishing treatments such as liquid cleaners and refresher products such as odour treatment agents as exemplified by Febreze® (P&G). Household cleaners may be in the form of cream cleaners, isotropic liquid cleaners, spray cleaners and pre-moistened surface cleaning wipes (reference can be made for example to WO91/08283, EP743280, WO96/34938, WO01/23510, and WO99/28428). Spray cleaners may be dispensed from a trigger sprayer or aerosol sprayer, as are well known in the art. An aerosol sprayer dispenses the product using propellant pressure, while a trigger sprayer dispenses the product by pumping it under manual actuation. A suitable aerosol dispenser may have a dip tube or bag on valve, according to US 2015/0108163 and/or US 2011/0303766. A suitable trigger sprayer is found in US 8,322,631.

Household products also include freshening composition which may be used in a device for the delivery of a volatile material to the atmosphere or on inanimate surfaces (e.g. fabric surfaces as a fabric refresher).

Household products further include absorbent articles, such as a sanitary product. Preferably said absorbent article comprises an absorbent core, and optionally a backsheet, topsheet, acquisition layer or outer wrapper, wherein the microcapsules of the invention are disposed on the absorbent core or between one or more of the optional layers. The absorbent article can be contained in a polybag or paper carton. The absorbent article may further comprise a lotion. The absorbent article may further comprise one or more adjunct ingredients selected from surfactants, inks, dyes, mineral oils, petrolatum, polysiloxanes, cyclodextrins, clays, silicates, aluminates, vitamins, isoflavones, flavones, metal oxides, short chain organic acids ($C_1$-$C_8$), triglycerides ($C_8$-$C_{22}$), and antioxidants. Laundry products include powdered laundry detergents, detergent tablets and bars, laundry detergent liquids include light duty liquids, heavy duty liquids, concentrated liquid detergents, non or low aqueous laundry liquids and more specialised cleaners for woollen or dark garments; and fabric softeners and pre- and post-wash treatments such as tumble drier sheets, ironing waters and wash additives such as the Lenor Unstoppables™ from Procter and Gamble, the Dr Beckman™ range of laundry additives Dr. Beckman International and the Vanish™ laundry additives from Reckitt Benckiser.

In one embodiment a laundry product is selected from the group consisting of a fabric softener, a fabric conditioner and a laundry detergent.

Fabric softeners and conditioners specifically include both conventional diluted (e.g. 2% to 8% by weight of softener in the product) liquid active concentration softeners and concentrated (e.g. 10% to 40% by weight of softener in the product) liquid active concentration softeners as well as fabric conditioners which may contain ingredients to protect colors or garment shape and appearance (reference can be made for example to US 6,335,315, US 5,674,832, US 5,759,990, US 5,877,145, US 5,574,179).

Laundry detergents, particularly liquid laundry detergents, specifically include light duty liquid detergents and heavy duty liquid detergents which may be structured multi-phase liquids or isotropic liquids and which may be aqueous or non-aqueous liquids. These liquids may be in bottles or unit dose sachets and they may optionally contain bleaching agents or enzymes (reference can be made for example to US 5,929,022, US 5,916,862, US 5,731,278, US 5,470,507, US 5,466,802, US 5,460,752, and US 5,458,810).

The products presently disclosed may contain water and/or surface active material, either as an emulsifier, if the product is an emulsion, or as a detergent active material if the product has some kind of cleaning function. In certain embodiments the concentration of surface active material in the product will be within the range 0.1-60% by weight; usually the level of surface active material will be 50% by weight or lower; for most products the level of surface active material will be 30% by weight or lower. On the other hand, the level of surface active material will usually be at least 0.1% by weight preferably greater than 1.0% and more preferably greater than 3.0% by weight. Certain product formulations are water sensitive (e.g. anti-perspirant, deodorant formulations, non-aqueous liquids packaged in water soluble polyvinyl alcohol films), and for these applications it may be desirable to spray dry the microcapsules to remove water, before the microcapsules are incorporated in the product formulation. For products which have a cleaning function it is likely the level of surface active material will be higher, typically greater than 10% by weight and preferably greater than 15% by weight. All percentages are expressed by weight over the weight of the product.

Examples of leave-on products containing emulsifiers are: hand and body lotions, make up removing lotions, skin creams, sunscreen products and sunless tanning products and domestic freshener sprays. Also included are articles of manufacture impregnated with liquids, for example pads or wipes impregnated with lotions for make-up application or removal, or to apply sunscreen compounds or sunless tanning agents, for personal cleansing e.g. as moist toilet tissue or baby wipes.

Examples of personal cleansing products containing detergents are: shampoos, body washes, liquid soaps. Some cleaning products may be considered leave on products even though they are used for cleansing if there is no rinsing or further cleaning action after use. Baby wipes are an example, although used for cleaning the liquid deposited on the skin is not removed by rinsing.

The non-rinsed cosmetic, toiletry and personal care compositions described herein can contain various emulsifiers which are useful for emulsifying the various components of the products. Suitable emulsifiers can include any of a wide variety of non-ionic, cationic, anionic, and zwitterionic surface active materials as disclosed in publications such as McCutcheon's, Detergents and Emulsifiers, North American Edition (1986), published by Allured Publishing Corporation and in the following patents: U.S. 5,011,681; U.S. 4,421,769; and U.S. 3,755,560.

Amounts of microcapsules dosed into liquid household, laundry, personal care and cosmetic products may vary depending on several aspects such as the desired microcapsule concentration, the proportion of fragrance within the microcapsules and the amount of fragrance necessary to create the olfactory effect desired. After removing all liquid components from a given product (i.e. measured as dry weight) the microcapsules may be present in an amount from 0.01 to 10% by weight, preferably from 0.05% to 2.5% by weight, more preferably from 0.1 to 1.25% by weight over the weight of the product. The dispersion of microcapsules may be incorporated at a suitable stage in the product manufacturing process but usually after any high shear mixing stage. If liquid at room temperature, it is preferable that the product into which the microcapsules are to be added has a viscosity greater than 20 MPa, for example greater than 100 MPa, or greater than 1,000 MPa, or even greater than 10,000 MPa, when measured at a low (e.g. 10rpm) spindle speed and at 25°C. If necessary, viscosity can be adjusted through the addition of conventional viscosity modifying agents. Suitable agents as well as equipment and conditions to measure the viscosity of a product are discussed in Rheology Modifiers Handbook Practical Uses and Applications by M R Rosen and D Braun published by William Andrew Publishing in 2000 with ISBN 978-0-8155-1441-1.

Further embodiments and advantages of the present invention will become apparent to a skilled reader in light of the examples provided below.

**Examples**

Capsule particle size measurement

Median volume diameter and span were measured with a laser diffraction/scattering particle size distribution analyzer (trade name: LA-950V2, manufactured by Horiba, Ltd.). The dispersant was 18 MΩ water. Several droplets of the emulsion or the capsule dispersion were poured into the flow cell unit until an acceptable level of laser light obscuration was achieved and triplicate measurements were then immediately performed. For the calculation of the particle size measurement, the refractive indexes were set at 1.33 (for the water dispersant), 1.47 (for the fragrances and the capsules). The median capsule diameter was measured as a particle size of 50% frequency (median size) on a volumetric basis. The span was calculated according to the following formula:

$$Span = \frac{D(v;0.9) - D(v;0.1)}{D(v;0.5)}$$

in which D(v; 0.9) is the particle size for 90% of the microcapsules by volume, D(v; 0.1) is the particle size for 10% of the microcapsules by volume and D(v; 0.5) is the median volume microcapsule size as previously defined.

Solid content measurement method

Approximately 2.5 g of dispersion were weighted in an aluminum weighing dish and dried during four hours at 105 °C in order to remove water. The weight of the dry sample was then determined at room temperature and compared to the weight of the dispersion.

Biodearadabilitv:

The following standards can be used to test the biodegradability of the polymer wall of the microcapsules of the invention: OECD 301B, OECD 301F, OECD 311, ISO 14852:2018, ASTM D5338, or ISO 14855. The polymer wall of the microcapsules of the invention display a biodegradation rate of least 5%, such as at least 10%, at least 20% or at least 30% after 28 days, when tested according to standard OECD 301F.

**Example 1: Composition of fragrance A (wt%)**

| | |
|---|---|
| Verdox (CAS N° 88 41 5) | 38.50 |
| Isobornyl acetate (CAS N° 125-12-2) | 25.00 |
| Dimethyl benzyl carbinyl acetate (CAS N° 151-05-3) | 14.00 |
| 2,4-ivy carbaldehyde (CAS N° 68039-49-6, 144046-32-2) | 6.50 |
| 2-methyl undecanal (CAS N° 110-41-8) | 4.50 |
| Ethyl 2-methylbutyrate (CAS N°7452-79-1) | 4.50 |
| Ethyl-2-methylpentanoate (CAS N° 39255-32-8) | 3.00 |
| 2-(2-(4-methyl-3-cyclohexenyl-1-yl)propyl)cyclopentanone (CAS N° 95962-14-4) | 2.50 |
| Delta damascone (CAS N° 57378-68-4) | 1.50 |

**Example 2: Synthesis of a dispersion of microcapsules**

*Preparation of reactants*

A 10% PVA [poly(vinyl alcohol)] aqueous solution was prepared by dissolving Selvol® 823, hydrolyzed to 87-89% (Sekisui), in water.

A dispersion of silica in water was prepared separately by stirring 1.06 g of Aerosil® R816 silica and 129 g of water containing 100 mg/L of sodium bicarbonate: during 5 min using a stirrer bar and then during 1 min at 3500 rpm using a high-shear mixer (e.g. Ystral X 10/20 E3-1050 W equipped with a Dispermix head of diameter 40/54 mm).

An oil phase was prepared by mixing 120 g of fragrance A and 20 g of trimethylolpropane triacrylate.

An aqueous solution of xylylene diamine was prepared by adding 27.2 g of diamine to 25 g of deionized water.

*Preparation of microcapsules dispersion*

The oil phase and the aqueous dispersion of silica were mixed together over 10 min with a stirrer bar and then at 10000 rpm for 1 min using the high-shear mixer, whereby an emulsion was obtained. This emulsion was placed into a sealed 500 mL-batch reactor equipped with a condenser, a thermometer, a bottom outlet valve and an anchor stirrer. 46.7 g of the PVA aqueous solution were then added to the emulsion, and the mixture was heated at 50°C under stirring (250 rpm). The aqueous solution of xylylene diamine was then added to the emulsion over 15 min, and the reaction was stirred (250 rpm) at 50°C for 2h. The resulting microcapsule dispersion was cooled to room temperature. The median volume diameter (D(v, 0.5)) and the span of the microcapsules were determined by laser diffraction, and the solid content of the dispersion was calculated. The results are shown in the table below.

| D(v, 0.5) - μm (Span) | Dry content (%) |
|---|---|
| 31.9 (0.652) | 44.5 |

**Example 3: olfactive test**

The microcapsule dispersion prepared in example 2 was diluted at 0.3% w/w in water. 35 droplets of this dispersion were deposited homogeneously on cotton terry towels (30 cm * 20 cm, about 50 g) prewashed with an unperfumed liquid detergent at 90°C. The terry towels were let dry overnight at room temperature. The intensity of the fragrance was assessed as a blind experiment by a panel of 5 trained assessors: each assessor was given a dried towel and assessed the intensity before and after rubbing the towel against hands. Scores were given on an interval scale from 0 (non-noticeable scent) to 5 (very strong scent). The average score before and after rubbing is shown in the table below.

| before rubbing | -after rubbing |
|---|---|
| 0.8 | 2.9 |

It can be seen from the above table that the encapsulated fragrance released upon rubbing the towel.

**Claims**

1. An aqueous dispersion of microcapsules, said microcapsules comprising a hydrophobic core and a shell, wherein said shell is formed of the reaction product of (i) at least one α,β-unsaturated carbonyl compound, (ii) at least one multifunctional amine, and (iii) solid colloidal particles.

2. The dispersion of microcapsules of claim 1 wherein the at least one α,β-unsaturated carbonyl compound is an anhydride, a multifunctionalacrylamide or a multifunctional methacrylamide.

3. The dispersion of microcapsules of claim 1, wherein the at least one α,β-unsaturated carbonyl compound is a multifunctional (meth)acrylate.

4. The dispersion of claim 3, wherein the multifunctional (meth)acrylate is an ester of (meth)acrylic acid with a linear or branched, alicyclic, aromatic or heterocyclic ($C_2$-$C_{24}$)alcohol, or with a ($C_2$-$C_{24}$)polyethylene glycol.

5. The aqueous dispersion of claim 4, wherein the multifunctional (meth)acrylate is selected from 1,4-butylene glycol diacrylate, ethylene glycol diacrylate, diethylene glycol diacrylate, triethylene glycol diacrylate, neopentylglycol diacrylate, triglycerol diacrylate, 1,6-hexane diol diacrylate, trimethylolpropane triacrylate, trimethylolpropane ethoxylate triacrylate, trimethylolpropane propoxylate triacrylate, pentaerythritol triacrylate, tris-2-hydroxyethyl isocyanurate triacrylate, glycerol triacrylate, pentaerythritol tetraacrylate, di-trimethylolpropane tetraacrylate, dipentaerythritol pentaacrylate, dipentaerythritol hexaacrylate, 1,3-butylene glycol dimethacrylate, 1,4-butylene glycol dimethacrylate, 1,2-propylene glycol dimethacrylate, 1,3-propylene glycol dimethacrylate, ethylene glycol dimethacrylate, diethylene glycol dimethacrylate, glycerol dimethacrylate, 1,6-hexane diol dimethacrylate, bisphenol A dimethacrylate, bisphenol A ethoxylate dimethacrylate, pentaerythritol trimethacrylate, glycerol trimethacrylate, trimethylolpropane trimethacrylate, Tris-2-hydroxyethyl isocyanurate trimethacrylate, ethoxylated pentaerythritol tetramethacrylate, Tris[2-(acryloyloxy)ethyl]isocyanurate, 2-[4,6-bis(2-propenoyloxyethyl)-1,3,5-triazin-2-yl]ethyl prop-2-enoate, and mixtures thereof.

6. The aqueous dispersion of claim 5, wherein the multifunctional (meth)acrylate is selected from 1,4-butylene glycol diacrylate, ethylene glycol diacrylate, trimethylolpropane triacrylate, glycerol triacrylate, pentaerythritol triacrylate, pentaerythritol tetraacrylate, di-trimethylolpropane tetraacrylate, 1,4-butylene glycol dimethacrylate, ethylene glycol dimethacrylate, trimethylolpropane trimethacrylate, glycerol trimethacrylate, and mixtures thereof.

7. The aqueous dispersion of any one of claims 1 to 6, wherein the multifunctional amine is a compound of formula (1):

$$R_1HN\text{-}A\text{-}NHR_2 \qquad (1)$$

where A is a direct bond, a branched or linear ($C_2$-$C_8$)alkylene, a ($C_6$-$C_{12}$)arylene; a group $-(CH_2)_m-(C_6-C_{12})$arylene-$(CH_2)_m$- where m is an integer from 1 to 3, or a group $-CH_2-(CH_2-NH-CH_2)_v-CH_2-$ in which v is an integer from 1 to 4; and $R_1$ and $R_2$ are each independently H or a ($C_1$-$C_4$)alkyl, or $R_1$ and $R_2$ are bound together to form o 6- or 7-membered ring.

**8.** The aqueous dispersion of any one of claims 1 to 6, wherein the multifunctional amine is a compound of formula (2):

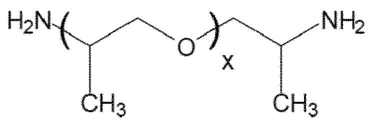

(2)

where x varies from 2 to 75.

**9.** The aqueous dispersion of any one of claims 1 to 6, wherein the multifunctional amine is a compound of formula (3):

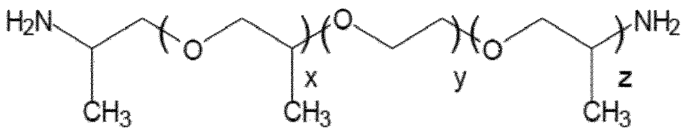

(3)

where x varies from 2 to 45 and (x+z) varies from 1 to 8.

**10.** The aqueous dispersion of any one of claims 1 to 6, wherein the multifunctional amine is a compound of formula (4):

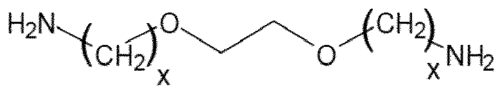

(4)

where x varies from 2 to 4.

**11.** The aqueous dispersion of any one of claims 1 to 6, wherein the multifunctional amine is a compound of formula (5):

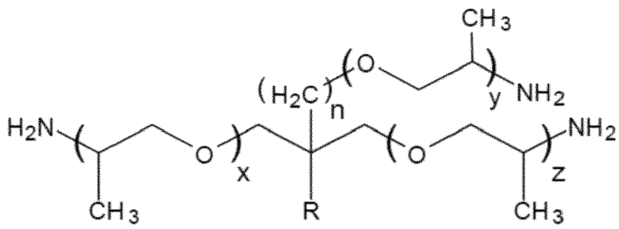

(5)

where R is H or $C_2H_5$, n is 0 or 1 and (x+y+z) varies from 5 to 90.

**12.** The aqueous dispersion of any one of claims 1 to 6, wherein the multifunctional amine is selected from 1,3-diaminobenzene, 1,4-diaminobenzene, 1,3-Bis(aminomethyl)benzene, 1,4-Bis(aminomethyl)benzene, triethylene tetramine, tetraethylene pentaamine, a compound of formula (2) where x =2 or 3, and a compound of formula (5) where (x+y+z) varies from 5 to 10.

**13.** The aqueous dispersion of any one of claims 1 to 12, wherein the solid colloidal particles are selected from particles of silica, particles of lithium magnesium sodium silicates, particles of nanocellulose and particles extracted from apple juice.

**14.** The aqueous dispersion of claim 13, wherein the solid colloidal particles are particles of silica.

**15.** The aqueous dispersion of any one of claims 1 to 11, wherein the core comprises a fragrance.

**16.** A product comprising the aqueous dispersion as defined in any one of claims 1 to 15.

**17.** The product of claim 16, which is a laundry product, a personal care product or a cosmetic product.

**18.** A process for the manufacture of an aqueous dispersion as defined in any one of claims 1 to 15, said process comprising:

(i) forming an oil-in-water emulsion wherein the oil phase comprises the constituent(s) of the core and the at least one $\alpha,\beta$-unsaturated carbonyl compound, and the aqueous phase comprises solid colloidal particles,
(ii) adding a polymeric stabilizer to the emulsion, and
(iii) adding an aqueous solution of the at least one multifunctional amine to the stabilized emulsion.

**19.** The process of claim 18, wherein the polymeric stabilizer has a molecular weight from about 10,000 g/mol to about 150,000 g/mol.

Europäisches Patentamt
European Patent Office
Office européen des brevets

# EUROPEAN SEARCH REPORT

Application Number
EP 20 30 5187

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2019/275490 A1 (BACHAWALA PRAVEEN [US]) 12 September 2019 (2019-09-12)<br>* paragraph [0038]; tables 12.4, entry AB * | 1-17<br>18,19 | INV.<br>B01J13/16<br>A61K8/11<br>A61K9/48<br>A61Q19/10<br>C11D3/50<br>C11D17/00 |
| X | YU FENGYANG ET AL: "Fabrication of compact poly(methyl methacrylate-co-butyl methacrylate-co-acrylic acid) microcapsules for electrophoretic displays by using emulsion droplets as templates", COLLOID & POLYMER SCIENCE, SPRINGER VERLAG, HEIDELBERG, DE, vol. 294, no. 8, 8 June 2016 (2016-06-08), pages 1359-1367, XP036000626, ISSN: 0303-402X, DOI: 10.1007/S00396-016-3901-Z [retrieved on 2016-06-08]<br>* under the heading: Transparent Microcapsules; figure 1 * | 1-19 | |
| X<br>A | WO 2011/056904 A1 (PROCTER & GAMBLE [US]; CHIEFFI ANDRE [GB] ET AL.) 12 May 2011 (2011-05-12)<br>* page 10, lines 19-20; example 1 * | 1-17<br>18,19 | TECHNICAL FIELDS SEARCHED (IPC)<br>B01J<br>C11D<br>A61Q<br>A61K |

The present search report has been drawn up for all claims

| Place of search | Date of completion of the search | Examiner |
|---|---|---|
| The Hague | 16 July 2020 | Mc Donnell, Shane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

1

EPO FORM 1503 03.82 (P04C01)

## ANNEX TO THE EUROPEAN SEARCH REPORT ON EUROPEAN PATENT APPLICATION NO. EP 20 30 5187

This annex lists the patent family members relating to the patent documents cited in the above-mentioned European search report. The members are as contained in the European Patent Office EDP file on
The European Patent Office is in no way liable for these particulars which are merely given for the purpose of information.

16-07-2020

| Patent document cited in search report | Publication date | Patent family member(s) | Publication date |
|---|---|---|---|
| US 2019275490 A1 | 12-09-2019 | US 2019275490 A1 | 12-09-2019 |
| | | WO 2019173062 A1 | 12-09-2019 |
| WO 2011056904 A1 | 12-05-2011 | AR 078889 A1 | 07-12-2011 |
| | | AR 078890 A1 | 07-12-2011 |
| | | BR 112012010650 A2 | 08-08-2017 |
| | | BR 112012010676 A2 | 24-09-2019 |
| | | BR 112012011580 A2 | 28-06-2016 |
| | | CA 2778251 A1 | 12-05-2011 |
| | | CN 102597203 A | 18-07-2012 |
| | | CN 102597205 A | 18-07-2012 |
| | | CN 102612553 A | 25-07-2012 |
| | | CN 105107438 A | 02-12-2015 |
| | | EP 2496678 A1 | 12-09-2012 |
| | | EP 2496681 A1 | 12-09-2012 |
| | | EP 2496682 A1 | 12-09-2012 |
| | | EP 3255135 A1 | 13-12-2017 |
| | | ES 2647570 T3 | 22-12-2017 |
| | | HK 1212281 A1 | 10-06-2016 |
| | | JP 6430282 B2 | 28-11-2018 |
| | | JP 2013509988 A | 21-03-2013 |
| | | JP 2015155540 A | 27-08-2015 |
| | | JP 2018172687 A | 08-11-2018 |
| | | MX 337136 B | 12-02-2016 |
| | | PL 2496681 T3 | 31-01-2018 |
| | | PL 3255135 T3 | 31-07-2019 |
| | | US 2011107524 A1 | 12-05-2011 |
| | | US 2011110993 A1 | 12-05-2011 |
| | | US 2011111999 A1 | 12-05-2011 |
| | | US 2014249069 A1 | 04-09-2014 |
| | | US 2015191682 A1 | 09-07-2015 |
| | | US 2016348043 A1 | 01-12-2016 |
| | | US 2016376535 A1 | 29-12-2016 |
| | | WO 2011056904 A1 | 12-05-2011 |
| | | WO 2011056934 A1 | 12-05-2011 |
| | | WO 2011056935 A1 | 12-05-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20150030557 A
- US 7069658 B
- US 6944952 B
- US 6594904 B
- US 6182365 B
- US 6185822 B
- US 6298558 B
- US 5113585 A
- US 6162423 A
- US 5968286 A
- US 5935561 A
- US 5932203 A
- US 5837661 A
- US 5776443 A
- US 5756436 A
- US 5661118 A
- US 5618523 A
- US 3697644 A
- US 4065398 A
- US 4387040 A
- US 4775582 A
- WO 0207701 A
- WO 2007069214 A
- WO 9516474 A
- WO 9108283 A
- EP 743280 A
- WO 9634938 A
- WO 0123510 A
- WO 9928428 A
- US 20150108163 A
- US 20110303766 A
- US 8322631 B
- US 6335315 B
- US 5674832 A
- US 5759990 A
- US 5877145 A
- US 5574179 A
- US 5929022 A
- US 5916862 A
- US 5731278 A
- US 5470507 A
- US 5466802 A
- US 5460752 A
- US 5458810 A
- US 5011681 A
- US 4421769 A
- US 3755560 A


### Non-patent literature cited in the description


- MICROENCAPSULATION: Methods and Industrial Applications. Marcel Dekker, Inc, 1996
- Kirk Othmer's Encyclopaedia of Chemical Technology
- **A D MCNAUGHT ; A WILKINSON.** IUPAC Compendium of Chemical Terminology. Blackwell Scientific Publications, 1997
- IUPAC Nomenclature of Organic Chemistry. Blackwell Scientific Publications, 1993
- Surface modification of inorganic nanoparticles by organic functional groups. Nanoparticle Technology handbook. 2007, 593-596
- **S. ARCTANDER.** *Perfume Flavors and Chemicals,* 1969, vol. I, II
- The Merck Index. Merck & Co., Inc,
- **E GUENTHER.** The Essential Oils. Van Nostrand, 1949
- **L HO TAN TAI.** Formulating Detergents and Personal Care Products A guide to Product Development. AOCS Press
- Surfactant Science Series Liquid Detergents. Marcel Dekker Inc, vol. 67
- Harry's Cosmeticology. CHS Press, 2000
- North American Edition. **MCCUTCHEON'S.** Detergents and Emulsifiers. Allured Publishing Corporation, 1986
- **M R ROSEN ; D BRAUN.** Rheology Modifiers Handbook Practical Uses and Applications. William Andrew Publishing, 2000